# EUROPEAN PATENT APPLICATION

(11) **EP 2 351 738 A1**
(43) Date of publication of application: **03.08.2011**
(21) Application number: 09816311.6
(22) Date of filing: 25.09.2009
(51) Int. Cl.: C07D 211/58, C07B 53/00, C07B 61/00

(54) **PROCESS FOR PRODUCING 1-SUBSTITUTED TRANS-4-(SUBSTITUTED AMINO)PIPERIDIN-3-OL**

(30) Priority: 29.09.2008 JP 2008249973; 16.02.2009 JP 2009032332
(71) Applicant: Sumitomo Chemical Company, Limited, Tokyo 104-8260 (JP)
(72) Inventor: AIKAWA, Toshiaki, Toyonaka-shi Osaka 560-0021 (JP); TOKUDA, Osamu, Nara-shi Nara 631-0026 (JP); IKEMOTO, Tetsuya, Toyonaka-shi Osaka 561-0874 (JP)
(74) Representative: Lumsden, Stuart Edward Henry
(86) International application number: PCT/JP2009/067203
(87) International publication number: WO 2010/035902

(57) **Abstract**

A process for producing a 1-substituted trans-4-(substituted amino)piperidin-3-ol represented by formula (III-1): 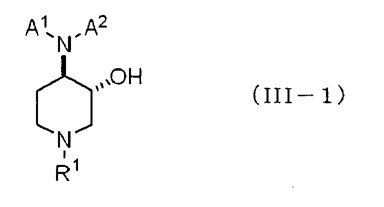 wherein R¹ represents an aromatic carbocyclic group, an alkyl group having 1 to 12 carbon atoms which may be substituted with one or more aromatic carbocyclic groups, an alkenyl group having 2 to 14 carbon atoms which may be substituted with one or more aromatic carbocyclic groups, or an alkynyl group having 2 to 12 carbon atoms which may be substituted with one or more aromatic carbocyclic groups, and the like,
which comprises a step of reacting a 1-substituted-3,4-epoxypiperidine represented by formula (I): 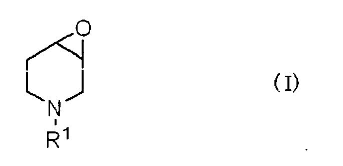 with an amine compound represented by formula (II) 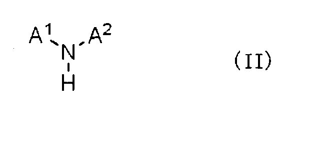 in the presence of an inorganic lithium salt. By utilizing the process, trans-4-aminopiperidin-3-ol compounds useful as various chemical products, such as medicine intermediates, can be produced.

## Description

### TECHNICAL FIELD

The present invention relates to a method for producing 1-substituted-trans-4-(substituted amino)piperidin-3-ol.

### BACKGROUND ART

A method for producing 1-alkoxycarbonyl-trans-4-benzylaminopiperidin-3-ol by reacting 3,4-epoxypiperidine in which a nitrogen atom on a piperidine ring is protected with a tert-butoxycarbonyl group with benzylamine is described in WO 2004/002490.

However, according to this method, there arises a problem that position selectivity of introduction of a benzylamino group is poor, and a 1-alkoxycarbonyl-trans-3-benzylaminopiperidin-4-ol that is an undesirable isomer is produced in the amount larger than that of 1-alkoxycarbonyl-trans-4-benzylaminopiperidin-3-ol.

### DISCLOSURE OF THE INVENTION

The present invention provides a method for producing a 1-substituted-trans-4-(substituted amino)piperidin-3-ol represented by formula (III-1): 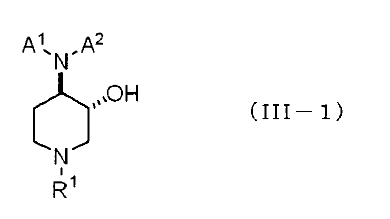 wherein R¹, A¹ and A² are as defined below,
which comprises a step of reacting a 1-substituted-3,4-epoxypiperidine represented by formula (I): 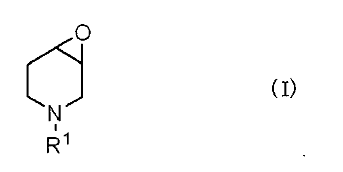 wherein R¹ represents an aromatic carbocyclic group, an alkyl group having 1 to 12 carbon atoms which may be substituted with one or more aromatic carbocyclic groups, an alkenyl group having 2 to 14 carbon atoms which may be substituted with one or more aromatic carbocyclic groups, or an alkynyl group having 2 to 12 carbon atoms which may be substituted with one or more aromatic carbocyclic groups, in which each aromatic carbocyclic group may be substituted with one or more substituents selected from the group consisting of an alkyl group having 1 to 12 carbon atoms, an alkoxy group having 1 to 12 carbon atoms, a halogen atom, a protected amino group and a protected hydroxyl group, with an amine compound represented by formula (II): 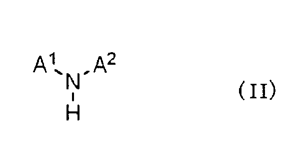 wherein A¹ represents an aromatic carbocyclic group, an alkyl group having 1 to 12 carbon atoms which may be substituted with one or more aromatic carbocyclic groups, an alkenyl group having 2 to 14 carbon atoms which may be substituted with one or more aromatic carbocyclic groups, or an alkynyl group having 2 to 12 carbon atoms which may be substituted with one or more aromatic carbocyclic groups, A² represents an alkyl group having 1 to 12 carbon atoms which may be substituted with one or more aromatic carbocyclic groups, an alkenyl group having 2 to 14 carbon atoms which may be substituted with one or more aromatic carbocyclic groups, an alkynyl group having 2 to 12 carbon atoms which may be substituted with one or more aromatic carbocyclic groups, or a hydrogen atom, or A¹ and A² represent a polymethylene group having 2 to 7 carbon atoms formed by combining together, in which each aromatic carbocyclic group may be substituted with one or more substituents selected from the group consisting of an alkyl group having 1 to 12 carbon atoms, an alkoxy group having 1 to 12 carbon atoms, a halogen atom, a protected amino group and a protected hydroxyl group, in the presence of an inorganic lithium salt.

In the above method, the inorganic lithium salt is preferably lithium halide or lithium perhalogenate, and more preferably lithium chloride or lithium perchlorate.

The 1-substituted-3,4-epoxypiperidine represented by formula (I) is preferably a compound represented by formula (I-A) : 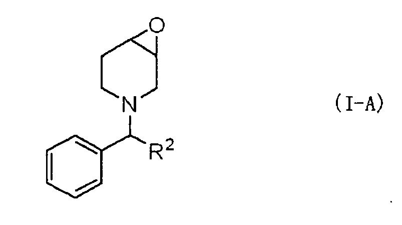 wherein R² represents an aralkyl group having 7 to 17 carbon atoms, an alkyl group having 1 to 11 carbon atoms, a phenyl group or a hydrogen atom.

The amine compound represented by formula (II) is preferably a compound represented by formula (II-A): 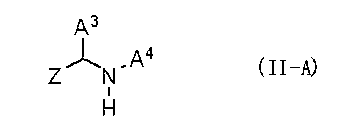 wherein A³ represents a hydrogen atom or an alkyl group having 1 to 11 carbon atoms, A⁴ represents a hydrogen atom, a benzyl group or an allyl group, and Z represents a phenyl group or a vinyl group, or a compound represented by formula (II-D): 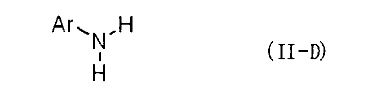 wherein Ar represents a phenyl group which may be substituted, and the substituent is one or more substituent(s) selected from the group consisting of an alkyl group having 1 to 12 carbon atoms, an alkoxy group having 1 to 12 carbon atoms, a halogen atom, a protected amino group and a protected hydroxyl group, and more preferably a compound represented by formula (II-B): 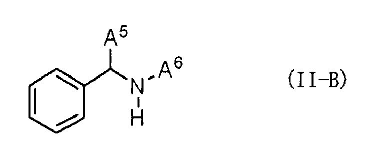 wherein A⁵ represents a hydrogen atom or an alkyl group having 1 to 11 carbon atoms, and A⁶ represents a hydrogen atom or a benzyl group, or a compound represented by formula (II-C) : 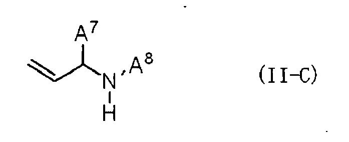 wherein A⁷ represents a hydrogen atom or an alkyl group having 1 to 11 carbon atoms, and A⁸ represents a hydrogen atom or an allyl group.

Further, the present invention provides a method for producing trans-4-aminopiperidin-3-ol represented by formula (IV): 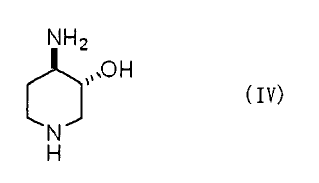 which comprises a step of reducing a 1-substituted-trans-4-(substituted amino)piperidin-3-ol represented by formula (III-B): 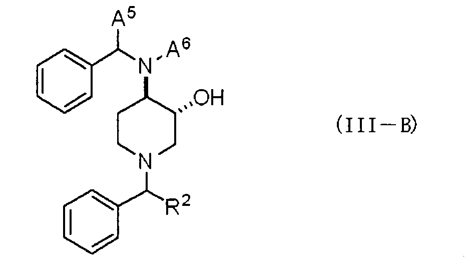 wherein R² represents an aralkyl group having 7 to 17 carbon atoms, an alkyl group having 1 to 11 carbon atoms or a phenyl group or hydrogen atom, A⁵ represents a hydrogen atom or an alkyl group having 1 to 11 carbon atoms, and A⁶ represents a hydrogen atom or a benzyl group.

Furthermore, the present invention provides a method for producing a trans-4-(protected amino)piperidin-3-ol represented by formula (VII): 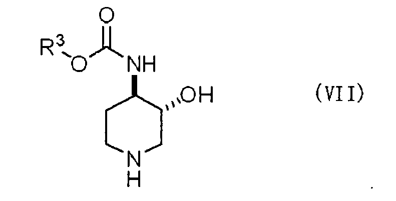 wherein R³ are as defined below, which comprises a step of removing a substituent on an amino group at the 4-position in a 1-substituted-trans-4-(substituted amino)piperidin-3-ol represented by formula (III-C): 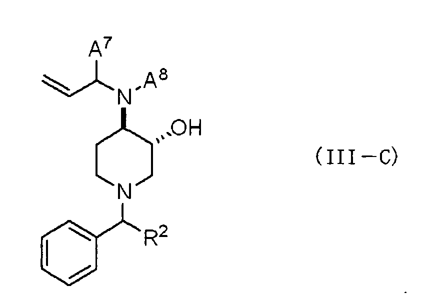 wherein R² represents an aralkyl group having 7 to 17 carbon atoms, an alkyl group having 1 to 11 carbon atoms, a phenyl group or a hydrogen atom, A⁷ represents a hydrogen atom or an alkyl group having 1 to 11 carbon atoms, and A⁸ represents a hydrogen atom or an allyl group, to obtain a 1-substituted-trans-4-aminopiperidin-3-ol represented by formula (V): 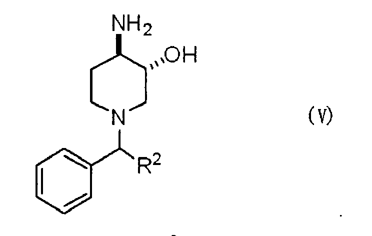 wherein R² are as defined above; a step of protecting an amino group at the 4-position in the 1-substituted-trans-4-aminopiperidin-3-ol represented by formula (V) to obtain a 1-substituted-trans-4-(protected amino)piperidin-3-ol represented by formula (VI): 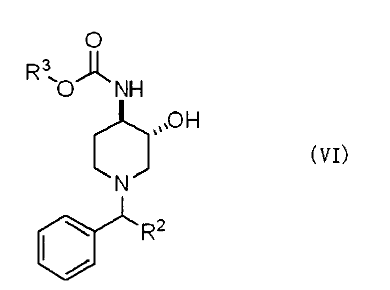 wherein R² is as defined above, and R³ represents an alkyl group having 1 to 12 carbon atoms; and a step of removing a substituent on a nitrogen atom contained in a piperidine ring in the 1-substituted-trans-4-(protected amino)piperidin-3-ol represented by formula (VI).

In the above method, R³ is preferably a tert-butyl group.

The present invention will be described in more detail below.

Examples of the aromatic carbocyclic group represented by R¹ in formula (I) include aromatic hydrocarbon groups having 6 to 10 carbon atoms, such as a phenyl group, a naphthyl group, and the aromatic carbocyclic group may have one or more substituents selected from the group consisting of an alkyl group having 1 to 12 carbon atoms, an alkoxy group having 1 to 12 carbon atoms, a halogen atom, a protected amino group and a protected hydroxyl group. Examples of the alkyl group having 1 to 12 carbon atoms include a methyl group, an ethyl group, a propyl group, an isopropyl group, a butyl group, an isobutyl group, a tert-butyl group, a pentyl group, a hexyl group, a heptyl group, an octyl group, a nonyl group, a decyl group, a undecyl group and a dodecyl group; examples of the alkoxy group having 1 to 12 carbon atoms include a methoxy group, an ethoxy group, a propoxy group and a butoxy group; examples of the halogen atom include a fluorine atom, a chlorine atom, a bromine atom and an iodine atom; examples of the protected amino group include an alkanoylamino group having 1 to 8 carbon atoms (for example, acetylamino group), a phenyl group which may have one or more substituents selected from the group consisting of an alkyl group having 1 to 8 carbon atoms, an alkoxy group having 1 to 8 carbon atoms, a halogen atom, a nitro group, a cyano group and a trifluoromethyl group on a benzene ring (for example, a benzoylamino group, a p-toluoylamino group, a 4-chlorobenzoylamino group, a 4-nitrobenzoylamino group, a 4-cyanobenzoylamino group, a 4-trifluoromethylbenzoylamino group, a 3,5-bis(trifluoromethyl)benzoylamino group), a phenoxycarbonylamino group which may have one or more substituents selected from the group consisting of alkoxycarbonylamino group having 1 to 8 carbon atoms (for example, a methoxycarbonylamino group, an ethoxycarbonylamino group, a t-butoxycarbonylamino group), an alkyl group having 1 to 8 carbon atoms, an alkoxy group having 1 to 8 carbon atoms, a halogen atom, a nitro group, a cyano group and a trifluoromethyl group on a benzene ring (for example, a phenoxycarbonylamino group, a 4-nitrophenoxycarbonylamino group) and a benzyloxycarbonylamino group which may have one or more substituents selected from the group consisting of an alkyl group having 1 to 8 carbon atoms, an alkoxy group having 1 to 8 carbon atoms, a halogen atom, a nitro group, a cyano group and a trifluoromethyl group on a benzene ring (for example, a benzyloxycarbonylamino group); and examples of the protected hydroxyl group include a benzoyloxy group which may have one or more substituents selected from the group consisting of an alkanoyloxy group having 1 to 8 carbon atoms (for example, an acetoxy group), an alkyl group having 1 to 8 carbon atoms, an alkoxy group having 1 to 8 carbon atoms, a halogen atom, a nitro group, a cyano group and a trifluoromethyl group on a benzene ring (for example, a benzoyloxy group, a 4-nitrobenzoyloxy group), a benzyloxy group which may have one or more substituents selected from the group consisting of an alkyl group having 1 to 8 carbon atoms, an alkoxy group having 1 to 8 carbon atoms, a halogen atom, a nitro group, a cyano group and a trifluoromethyl group on a benzene ring (for example, a benzyloxy group, a 4-methoxybenzyloxy group, a 4-nitrobenzyloxy group), an alkoxymethoxy group whose alkoxy group has 1 to 8 carbon atoms (for example, a methoxymethoxy group), a 1-(alkoxy)ethoxy group whose alkoxy group has 1 to 8 carbon atoms (for example, a 1-(ethoxy)ethoxy group), a 2-tetrahydropyranyloxy group, and a trialkylsilyloxy group in which each alkyl group has 1 to 8 carbon atoms (for example, a t-butyldimethylsilyloxy group).

Examples of the alkyl group having 1 to 12 carbon atoms which may be substituted with an aromatic carbocyclic group represented by R¹ include a methyl group, an ethyl group, a propyl group, an isopropyl group, a butyl group, an isobutyl group, a tert-butyl group, a pentyl group, a hexyl group, a heptyl group, an octyl group, a nonyl group, a decyl group, an undecyl group and a dodecyl group, and these alkyl groups having 1 to 12 carbon atoms may have one or more aromatic carbocyclic groups. Examples of the aromatic carbocyclic group are the groups shown as the aromatic carbocyclic group represented by R¹, and the aromatic carbocyclic group may be substituted with the same as in R¹. Examples of the alkyl group having 1 to 12 carbon atoms substituted with an aromatic carbocyclic group include a 1-phenylethyl group, a 2-phenylethyl group, a 1-naphthylethyl group, a 1-phenylpropyl group, a 2-phenylpropyl group, a 3-phenylpropyl group and a diphenylmethyl group.

Examples of the alkenyl group having 2 to 14 carbon atoms which may be substituted with an aromatic carbocyclic group represented by R¹ include a vinyl group, a 1-propenyl group, an allyl group, a butenyl group, a butadienyl group, a pentenyl group, a hexenyl group, a heptenyl group and an octenyl group, and these alkenyl group having 2 to 14 carbon atoms may have one or more aromatic carbocyclic groups. Examples of the aromatic carbocyclic group are the groups shown as the aromatic carbocyclic group represented by R¹, and the aromatic carbocyclic group may be substituted with the same as in R¹. Examples of the alkenyl group having 2 to 14 carbon atoms substituted with the aromatic carbocyclic group include a cinnamyl group and a styryl group.

Examples of the alkynyl group having 2 to 12 carbon atoms which may be substituted with an aromatic carbocyclic group represented by R¹ include an ethynyl group, a propynyl group, a butynyl group, a pentynyl group, a hexynyl group, a heptynyl group and a octynyl group, and these alkynyl groups having 2 to 12 carbon atoms may have one or more aromatic carbocyclic groups. Examples of the aromatic carbocyclic group are the groups shown as the aromatic carbocyclic group represented by R¹, and the aromatic carbocyclic group may be substituted with the same as in R¹. Examples of the alkynyl group having 2 to 12 carbon atoms substituted with the aromatic carbocyclic group include a 3-phenyl-2-propynyl group.

R¹ is preferably an alkyl group having 1 to 12 carbon atoms substituted with an aromatic carbocyclic group from the viewpoint of easiness of elimination, more preferably an alkyl group having 1 to 12 carbon atoms which has an aromatic group at the 1-position of an alkyl group, such as a benzyl group or a 1-phenylethyl group, and still more preferably a benzyl group.

Examples of the 1-substituted-3,4-epoxypiperidine represented by formula (I) (hereinafter abbreviated to compound (I)) include 3-methyl-7-oxa-3-azabicyclo[4.1.0]heptane, 3-ethyl-7-oxa-3-azabicyclo[4.1.0]heptane, 3-benzyl-7-oxa-3-azabicyclo[4.1.0]heptane, 3-(1-phenylethyl)-7-oxa-3-azabicyclo[4.1.0]heptane, 3-(2-phenylethyl)-7-oxa-3-azabicyclo[4.1.0]heptane, 3-propyl-7-oxa-3-azabicyclo[4.1.0]heptane, 3-isopropyl-7-oxa-3-azabicyclo[4.1.0]heptane, 3-butyl-7-oxa-3-azabicyclo[4.1.0]heptane, 3-(1-phenylpropyl)-7-oxa-3-azabicyclo[4.1.0]heptane, 3-(2-phenylpropyl)-7-oxa-3-azabicyclo[4.1.0]heptane, 3-(3-phenylpropyl)-7-oxa-3-azabicyclo[4.1.0]heptane, 3-(1-phenyl-1-methylethyl)-7-oxa-3-azabicyclo[4.1.0]heptane, 3-(1,1-diphenylmethyl)-7-oxa-3-azabicyclo[4.1.0]heptane, 3-butyl-7-oxa-3-azabicyclo[4.1.0]heptane and 3-isobutyl-7-oxa-3-azabicyclo[4.1.0]heptane. Compound (I) may be a racemic form or an optically active substance. Compound (I) is preferably a compound capable of easily removing a substituent R¹, for example, 3-benzyl-7-oxa-3-azabicyclo[4.1.0]heptane, 3-(1-phenylethyl)-7-oxa-3-azabicyclo[4.1.0]heptane and the like, and particularly preferably 3-benzyl-7-oxa-3-azabicyclo[4.1.0]heptane. Compound (I) can be produced in accordance with a known method, for example, described in Chem. Pharm. Bull., 29, 3026 (1981).

Examples of the aromatic carbocyclic group represented by A¹, as well as the alkyl group having 1 to 12 carbon atoms which may be substituted with one or more aromatic carbocyclic groups, the alkenyl group having 2 to 14 carbon atoms which may be substituted with one or more aromatic carbocyclic groups, and the alkynyl group having 2 to 12 carbon atoms which may be substituted with one or more aromatic carbocyclic groups represented by A¹ and A² in formula (II) include the same groups as those shown for R¹.

Examples of the polymethylene group having 2 to 7 carbon atoms represented by A¹ and A² formed by combining together in formula (II) include an ethylene group, a trimethylene group, a tetramethylene group, a pentamethylene group, a hexamethylene group and a heptamethylene group.

Examples of the amine compound represented by formula (II) (hereinafter abbreviated to compound (II)) include methylamine, ethylamine, benzylamine, phenylethylamine, propylamine, isopropylamine, butylamine, vinylamine, 1-propenylamine, allylamine, 1-butenylamine, 2-butenylamine, 3-butenylamine, 1,3-butadienylamine, cinnamylamine, styrylamine, ethynylamine, 2-propynylamine, 3-phenyl-2-propynylamine, dimethylamine, N-methylethylamine, N-methylbenzylamine, N-methyl-1-phenylethylamine, N-methyl-2-phenylethylamine, N-methylallylamine, N-methyl-cinnamylamine, N-methyl-2-propynylamine, N-methyl-3-phenyl-2-propynylamine, diethylamine, N-ethylbenzylamine, N-ethyl-1-phenylethylamine, N-ethyl-2-phenylethylamine, N-ethylallylamine, N-ethyl-cinnamylamine, N-ethyl-2-propynylamine, N-ethyl-3-phenyl-2-propynylamine, dibenzylamine, N-benzyl-1-phenylethylamine, N-benzyl-2-phenylethylamine, N-benzylallylamine, N-benzyl-cinnamylamine, N-benzyl-2-propynylamine, N-benzyl-3-phenyl-2-propynylamine, aniline, anisidine, aziridine, trimethyleneimine, pyrrolidine , piperidine , hexamethyleneimine and heptamethyleneimine. When compound (II) has an asymmetric carbon atom, a racemic form can be used and an optically active substance can also be used. It is also possible to use, as compound (II), a commercially available compound, and those prepared by any known method.

Examples of the inorganic lithium salt include lithium chloride, lithium bromide, lithium iodide, lithium perchlorate, lithium periodate, lithium carbonate, lithium sulfate and lithium phosphate. Among these inorganic lithium salts, lithium halides such as lithium chloride, lithium bromide and lithium iodide; and lithium perhalogenates such as lithium perchlorate and lithium periodate are preferable, and lithium chloride and lithium perchlorate are more preferable. It is also possible to use, as the inorganic lithium salt, a commercially available inorganic lithium salt, and those prepared by any known method.

In the reaction of compound (I) with compound (II) in the presence of an inorganic lithium salt, the use amount of compound (II) is preferably 1 mol or more based on 1 mol of compound (I). There is no particular limitation on the upper limit. For example, when compound (II) is liquid under the reaction conditions, the compound can also be used in an excess amount while serving also as a solvent. The use amount of compound (II) is more preferably from 1 to 5 mol, and still more preferably from 1 to 2 mol, based on 1 mol of compound (I) from the viewpoint of economical efficiency. The use amount of the inorganic lithium salt is preferably from 0.1 to 10 mol, more preferably from 0.3 to 2 mol, and still more preferably from 0.6 to 1.5 mol, based on 1 mol of compound (I).

This reaction is preferably carried out in the presence of a solvent. The solvent may be inert to the reaction, and examples thereof include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, tert-butylcyclohexane and petroleum ether; aromatic solvents such as benzene, toluene, ethylbenzene, isopropylbenzene, tert-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene and 1,2,4-trichlorobenzene; ether solvents such as tetrahydrofuran, methyltetrahydrofuran, diethyl ether, dipropyl ether, diisopropyl ether, dibutyl ether, dipentyl ether, dihexyl ether, diheptyl ether, dioctyl ether, tert-butyl methyl ether, cyclopentyl methyl ether, 1,2-dimethoxyethane, diethylene glycol dimethyl ether, anisole and diphenyl ether; alcohol solvents such as methanol, ethanol, 1-propanol, 2-propanol, 1-butanol, isobutyl alcohol, tert-butyl alcohol, 1-pentanol, 2-pentanol, isopentyl alcohol, 1-hexanol, 2-hexanol, isohexyl alcohol, 1-heptanol, 2-heptanol, 3-heptanol, isopeptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-tert-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether and diethylene glycol mono-tert-butyl ether; ester solvents such as ethyl acetate, propyl acetate, isopropyl acetate, butyl acetate, isobutyl acetate, tert-butyl acetate, amyl acetate and isoamyl acetate; nitrile solvents such as acetonitrile and propionitrile; aprotic polar solvents such as dimethyl sulfoxide, sulfolane, N,N-dimethylformamide, N,N-dimethylacetamide, N,N-dimethylpropionamide, N-methylpyrrolidone, γ-butyrolactone, dimethyl carbonate, diethyl carbonate, ethylene carbonate, propylene carbonate, 1,3-dimethyl-2-imidazolidinone and 1,3-dimethyl-3,4,5,6-tetrahydro-2(1H)-pyridinone; water; and mixtures thereof. Among these solvents, nitrile solvents are preferable, and acetonitrile is more preferable. The use amount of the solvent is preferably from 1 to 50 L, and more preferably from 2 to 15 L, based on 1 kg of compound (I).

The reaction temperature is preferably within a range from -20 to 100°C, and more preferably from 10 to 60°C. The reaction time varies depending on the reaction temperature and the use amount of the reaction reagent and the solvent, and is preferably from 1 to 24 hours. The degree of proceeding of the reaction can be confirmed by analysis means such as thin-layer chromatography, gas chromatography and high-performance liquid chromatography.

There is no particular limitation on the order of mixing reaction reagents. For example, mixing can be carried out by a method of adding compound (II) and an inorganic lithium salt to compound (I) or a solution thereof in any order.

The 1-substituted-trans-4-(substituted amino)piperidin-3-ol represented by formula (III-1) (hereinafter abbreviated to compound (III-1)) is contained in the mixture obtained after completion of the reaction as a main product. Although the 1-substituted-trans-3-(substituted amino)piperidin-4-ol represented by formula (III-2): 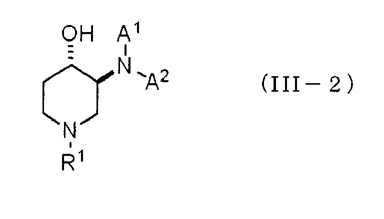 wherein R¹, A¹ and A² are as defined above (hereinafter abbreviated to compound (III-2)) may be sometimes contained as a by-product, a production ratio of them is within a range from compound (III-1):compound (III-2) = 80:20 to 100:0, or 90:10 to 100:0.

When the mixture obtained after completion of the reaction, which contains compound (III-1), is subjected to post-treatments such as filtration, extraction, washing with water and then subjected to isolation treatments such as distillation and crystallization, compound (III-1) can be extracted alone or a mixture with compound (III-2). At this time, compound (III-1) may be extracted as a salt of any acid such as hydrochloric acid, benzoic acid or tartaric acid. The thus extracted compound (III-1) or a salt thereof may be purified by purification treatments, for example, recrystallization; extraction and purification; distillation; treatments of absorption to activated carbon, silica, alumina and the like; and chromatography such as silica gel column chromatography.

Examples of compound (III-1) include trans-1-methyl-4-(methylamino)piperidin-3-ol, trans-1-ethyl-4-(methylamino)piperidin-3-ol, trans-1-benzyl-4-(methylamino)piperidin-3-ol, trans-1-benzyl-4-(ethylamino)piperidin-3-ol, trans-1-benzyl-4-(benzylamino)piperidin-3-ol, trans-1-benzyl-4-(diethylamino)piperidin-3-ol, trans-1-benzyl-4-(dibenzylamino)piperidin-3-ol, trans-1-benzyl-4-(phenylamino)piperidin-3-ol, trans-4-(benzylamino)-1-(1-phenylethyl)piperidin-3-ol, trans-4-(allylamino)-1-benzylpiperidin-3-ol, trans-4-(diallylamino)-1-benzylpiperidin-3-ol, trans-4-(benzylamino)-1-propylpiperidin-3-ol, trans-1-benzyl-4-(phenylamino)piperidin-3-ol, trans-1-benzyl-4-(pyrrolidin-1-yl)piperidin-3-ol and trans-1-benzyl-4-(piperidin-1-yl)piperidin-3-ol. When at least one of compound (I) and compound (II) is an optically active substance, the obtained compound (III-1) is also an optically active substance. Also, when compound (III-1) is a trans form, it means that a group represented by the formula: -NA¹A² and a hydroxyl group are mutually on opposite sides of the piperidine ring. Although the compound in which a group represented by the formula: -NA¹A² and a hydroxyl group are mutually on the same side of the piperidine ring is a cis form, it is preferable in the present invention that the cis form is not produced.

In the present reaction, when a compound represented by formula (I-A) (hereinafter abbreviated to compound (I-A)) is used as compound (I) and a compound represented by formula (II-A) (hereinafter abbreviated to compound (II-A)) is used as compound (II), it is possible to obtain, as compound (III-1), a compound represented by the formula (III-A) : 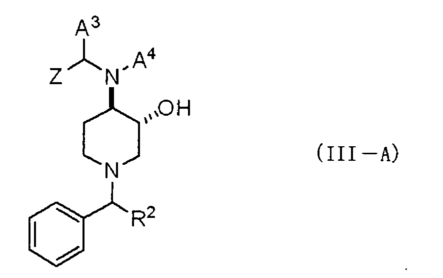 wherein R², A³, A⁴ and Z are respectively as defined above (hereinafter abbreviated to compound (III-A)).

Examples of the alkyl group having 1 to 11 carbon atoms represented by R² in formula (I-A) include a methyl group, an ethyl group, a propyl group, an isopropyl group, a butyl group, an isobutyl group, a tert-butyl group, a pentyl group, a hexyl group, a heptyl group, an octyl group, a nonyl group, a decyl group and an undecyl group. The aralkyl group having 7 to 17 carbon atoms is a group having one or more aryl groups such as a phenyl group and a naphthyl group on these alkyl groups having 1 to 11 carbon atoms, and examples thereof include a benzyl group, a 1-phenylethyl group, a 2-phenylethyl group, a 1-naphthylethyl group, a 1-phenylpropyl group, a 2-phenylpropyl group, a 3-phenylpropyl group, a 1-phenyl-1-methylethyl group, a 1-phenylbutyl group, a 2-phenylbutyl group, a 3-phenylbutyl group, a 4-phenylbutyl group and a 1-phenyl-1-methylpropyl group. R² is preferably a hydrogen atom.

Examples of compound (I-A) include 3-benzyl-7-oxa-3-azabicyclo[4.1.0]heptane, 3-(1-phenylethyl)-7-oxa-3-azabicyclo[4.1.0]heptane, 3-(1-phenylpropyl)-7-oxa-3-azabicyclo[4.1.0]heptane, 3-(1-phenylbutyl)-7-oxa-3-azabicyclo[4.1.0]heptane, 3-(1-phenyl-2-methylpropyl)-7-oxa-3-azabicyclo[4.1.0]heptane and 3-(1,3-diphenylpropyl)-7-oxa-3-azabicyclo[4.1.0]heptane. From the viewpoint of easiness of deprotection described hereinafter, 3-benzyl-7-oxa-3-azabicyclo[4.1.0]heptane is preferable. Compound (I-A) may be a racemic form or an optically active form.

Examples of the alkyl group having 1 to 11 carbon atoms represented by A³ in formula (II-A) include a methyl group, an ethyl group, a benzyl group, a propyl group, an isopropyl group, a butyl group, an isobutyl group, a tert-butyl group, a pentyl group, a hexyl group, a heptyl group, an octyl group, a nonyl group, a decyl group and an undecyl group.

Examples of compound (II-A) include benzylamine, 1-phenylethylamine, 1-phenylpropylamine, 1-phenylbutylamine, 1-phenyl-2-methylpropylamine, 1-phenyl-2-methylbutylamine, dibenzylamine, benzyl(1-phenylethyl)amine, benzyl(1-phenylpropyl)amine, benzyl(1-phenylbutyl)amine, benzyl(1-phenyl-2-methylpropyl)amine, benzyl(1-phenyl-2-methylbutyl)amine, allylamine and diallylamine. Compound (II-A) may be a racemic form or an optically active form.

Examples of compound (III-A) include trans-1-benzyl-4-(benzylamino)piperidin-3-ol, trans-4-(allylamino)-1-benzylpiperidin-3-ol, trans-1-benzyl-4-[(1-phenylethyl)amino]piperidin-3-ol, trans-1-benzyl-4-[(1-phenyl-2-methylpropyl)amino]piperidin-3-ol, trans-1-benzyl-4-[(1-phenylbutyl)amino]piperidin-3-ol, trans-1-benzyl-4-(dibenzylamino)piperidin-3-ol, trans-4-(benzylamino)-1-(1-phenylethyl)piperidin-3-ol, trans-4-(allylamino)-1-(1-phenylethyl)piperidin-3-ol, trans-1-(1-phenylethyl)-4-[(1-phenylethyl)amino]piperidin-3-ol, trans-1-(1-phenylethyl)-4-[(1-phenyl-2-methylpropyl)amino]piperidin-3-ol, trans-4-[(1-phenylbutyl)amino]-1-(1-phenylethyl)piperidin-3-ol, trans-4-(dibenzylamino)-1-(1-phenylethyl)piperidin-3-ol, trans-4-(benzylamino)-1-(1-phenylpropyl)piperidin-3-ol, trans-4-(allylamino)-1-(1-phenylpropyl)piperidin-3-ol, trans-4-[(1-phenylethyl)amino]-1-(1-phenylpropyl)piperidin-3-ol, trans-4-[(1-phenyl-2-methylpropyl)amino]-1-(1-phenylpropyl)piperidin-3-ol, trans-4-[(1-phenylbutyl)amino]-1-(1-phenylpropyl)piperidin-3-ol and trans-4-(dibenzylamino)-1-(1-phenylpropyl)piperidin-3-ol. When at least one of compounds (I-A) and (II-A) is an optically active substance, the obtained compound (III-A) is also the optically active substance.

In the present reaction, when compound (I-A) is used as compound (I) and a compound represented by formula (II-B) (hereinafter abbreviated to compound (II-B)) is used as the compound (II), it is possible to obtain, as compound (III-1), a compound represented by formula (III-B): 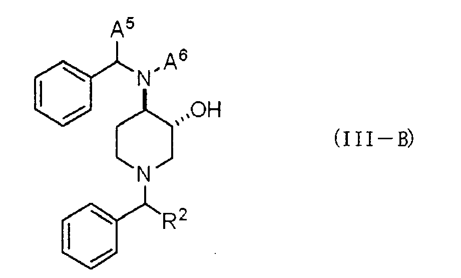 wherein A⁵, A⁶ and R² are respectively as defined above (hereinafter abbreviated to compound (III-B)).

Examples of the alkyl group having 1 to 11 carbon atoms represented by A⁵ in formula (II-B) include a methyl group, an ethyl group, a propyl group, an isopropyl group, a butyl group, an isobutyl group, a tert-butyl group, a pentyl group, a hexyl group, a heptyl group, an octyl group, a nonyl group, a decyl group and an undecyl group.

Examples of compound (II-B) include benzylamine, 1-phenylethylamine, 1-phenylpropylamine, 1-phenylbutylamine, 1-phenyl-2-methylpropylamine, 1-phenyl-2-methylbutylamine, dibenzylamine, benzyl(1-phenylethyl)amine, benzyl(1-phenylpropyl)amine, benzyl(1-phenylbuyl)amine, benzyl(1-phenyl-2-methylpropyl)amine and benzyl(1-phenyl-2-methylbutyl)amine. The compound (II-B) may be a racemic form or an optically active form.

Examples of compound (III-B) include trans-1-benzyl-4-(benzylamino)piperidin-3-ol, trans-1-benzyl-4-[(1-phenylethyl)amino]piperidin-3-ol, trans-1-benzyl-4-[(1-phenyl-2-methylpropyl)amino]piperidin-3-ol, trans-1-benzyl-4-[(1-phenylbutyl)amino]piperidin-3-ol, trans-1-benzyl-4-(dibenzylamino)piperidin-3-ol, trans-4-(benzylamino)-1-(1-phenylethyl)piperidin-3-ol, trans-1-(1-phenylethyl)-4-[(1-phenylethyl)amino]piperidin-3-ol, trans-1-(1-phenylethyl)-4-[(1-phenyl-2-methylpropyl)amino]piperidin-3-ol, trans-4-[(1-phenylbutyl)amino]-1-(1-phenylethyl)piperidin-3-ol, trans-4-(dibenzylamino)-1-(1-phenylethyl)piperidin-3-ol, trans-4-(benzylamino)-1-(1-phenylpropyl)piperidin-3-ol, trans-4-[(1-phenylethyl)amino]-1-(1-phenylpropyl)piperidin-3-ol, trans-4-[(1-phenyl-2-methylpropyl)amino]-1-(1-phenylpropyl)piperidin-3-ol, trans-4-[(1-phenylbutyl)amino]-1-(1-phenylpropyl)piperidin-3-ol and trans-4-(dibenzylamino)-1-(1-phenylpropyl)piperidin-3-ol. When at least one of compounds (I-A) and (II-B) is an optically active substance, the obtained compound (III-B) is also the optically active substance.

The step of reducing a compound (III-B) to obtain trans-4-aminopiperidin-3-ol represented by formula (IV) (hereinafter abbreviated to compound (IV)) (hereinafter may also be referred to as a reduction step) will be described below. In compound (III-B), R² is preferably a hydrogen atom, and both A⁵ and A⁶ are preferably hydrogen atoms.

As compound (III-B), the above mixture obtained after completion of the reaction may be used as it is, or may be used after the post-treatment. Further, the isolated compound (III-B) or a salt thereof may be used, or the purified compound (III-B) or a salt thereof may be used.

The reduction step can be carried out by any known method capable of deprotecting a benzyl protected amino group. Examples of the method include a method of reacting compound (III-B) with hydrogen in the presence of palladium on carbon, a method of reacting compound (III-B) with hydrogen in the presence of palladium hydroxide, and a method of reacting compound (III-B) with sodium in liquid ammonia, and preferable method is a method of reacting compound (III-B) with hydrogen in the presence of palladium on carbon.

Palladium on carbon may be a wet product or a dry product. The content of palladium atom is preferably from 0.5 to 50% by weight, and more preferably from 5 to 20% by weight. It is also possible to use, as palladium on carbon, commercially available palladium on carbon, and those prepared by any known method. The use amount of the palladium on carbon is usually within a range from 0.1 to 50 g, and preferably from 1 to 20 g, in terms of the palladium atom based on 1 kg of compound (III-B).

Palladium supported on carbon is usually zero-valent and when a di- or tetra-valent palladium compound is supported, it is preferably used after reducing to zero valence by a conventional method.

It is also possible to use, as hydrogen, a commercially available hydrogen gas, and those generated by any known method. The hydrogen pressure during the reaction is usually from 0.1 to 5 MPa, and preferably from 0.1 to 1 MPa. It is also possible to use as a mixed gas with an inert gas such as nitrogen or argon. In that case, the hydrogen partial pressure during the reaction is the same as the above hydrogen pressure.

The reaction of compound (III-B) with hydrogen is preferably carried out in the presence of a solvent. The solvent is preferably inert to the reaction, and examples thereof include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, tert-butylcyclohexane and petroleum ether; ether solvents such as tetrahydrofuran, methyltetrahydrofuran, diethyl ether, dipropyl ether, diisopropyl ether, dibutyl ether, dipentyl ether, dihexyl ether, diheptyl ether, dioctyl ether, tert-butyl methyl ether, cyclopentyl methyl ether, 1,2-dimethoxyethane and diethylene glycol dimethyl ether; alcohol solvents such as methanol, ethanol, 1-propanol, 2-propanol, 1-butanol, isobutyl alcohol, tert-butyl alcohol, 1-pentanol, 2-pentanol, isopentyl alcohol, 1-hexanol, 2-hexanol, isohexyl alcohol, 1-heptanol, 2-heptanol, 3-heptanol, isopentyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-tert-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether and diethylene glycol mono-tert-butyl ether; ester solvents such as ethyl acetate, propyl acetate, isopropyl acetate, butyl acetate, isobutyl acetate, tert-butyl acetate, amyl acetate and isoamyl acetate; aprotic polar solvents such as dimethyl sulfoxide, sulfolane, N,N-dimethylformamide, N,N-dimethylacetamide, N,N-dimethylpropionamide, N-methylpyrrolidone, γ-butyrolactone, dimethyl carbonate, diethyl carbonate, ethylene carbonate, propylene carbonate, 1,3-dimethyl-2-imidazolidinone and 1,3-dimethyl-3,4,5,6-tetrahydro-2(1H)-pyridinone; water; and mixtures thereof. Among these solvents, alcohol solvents are preferable, and ethanol is more preferable. The use amount of the solvent is preferably from 1 to 50 L, and more preferably from 2 to 15 L, based on 1 kg of compound (III-B).

The reaction temperature is preferably within a range from 0 to 100°C, and more preferably from 20 to 70°C. The reaction time varies depending on the reaction temperature, the reaction reagent, the use amount of the solvent, the hydrogen pressure and the like, and is preferably from 1 to 24 hours. The degree of proceeding of the reaction can be confirmed by analysis means such as thin-layer chromatography, gas chromatography and high-performance liquid chromatography.

There is no particular limitation on the order of mixing reaction reagents. For example, mixing can be carried out by a method of mixing a compound (III-B) or a solution thereof with palladium on carbon and adding hydrogen to the obtained mixture, or a method of adding compound (III-B) to palladium on carbon under a hydrogen atmosphere. A method of mixing a solution of compound (III-B) with palladium on carbon and adding hydrogen to the obtained mixture is preferable.

Compound (IV) is contained in the mixture obtained after completion of the reaction and, for example, when the mixture is subjected to post-treatments such as filtration, extraction, washing with water and the like and then subjected to isolation treatments such as distillation and crystallization, compound (IV) can be extracted. At this time, compound (IV) may be isolated as a salt of any acid such as hydrochloric acid, benzoic acid or tartaric acid. The isolated compound (IV) or a salt thereof can be purified by purification treatments such as recrystallization; extraction and purification; distillation; treatments of absorption to activated carbon, silica, alumina and the like; and chromatography such as silica gel column chromatography. When using, as the compound (III-B), an optically active substance whose optical activity is attributed to an asymmetric carbon on a piperidine ring, the obtained compound (IV) is also the optically active substance.

In the reaction of compound (I) with compound (II), when compound (I-A) is used as compound (I) and a compound represented by formula (II-C) (hereinafter abbreviated to compound (II-C)) is used as compound (II), compound (III-C) is obtained as compound (III-1). In formula (I-A), R² is preferably a hydrogen atom. In formula (II-C), examples of the alkyl group having 1 to 11 carbon atoms represented by A⁷ include a methyl group, an ethyl group, a benzyl group, a propyl group, a isopropyl group, a butyl group, an isobutyl group, a tert-butyl group, a pentyl group, a hexyl group, a heptyl group, an octyl group, a nonyl group, a decyl group and an undecyl group. Both A⁷ and A⁸ are preferably hydrogen atoms.

Examples of compound (III-C) include trans-4-(allylamino)-1-benzylpiperidin-3-ol, trans-4-(allylamino)-1-(1-phenylethyl)piperidin-3-ol and trans-4-(allylamino)-1-(1-phenylpropyl)piperidin-3-ol. When at least one of compounds (I-A) and (II-C) is an optically active substance, the obtained compound (III-C) is also the optically active substance.

The step of removing a substituent on an amino group at the 4-position in compound (III-C) to obtain a 1-substituted-trans-4-aminopiperidin-3-ol represented by formula (V) (hereinafter abbreviated to compound (V)); the step of protecting an amino group at the 4-position in compound (V) to obtain a 1-substituted-trans-4-(protected amino)piperidin-3-ol represented by formula (VI) (hereinafter abbreviated to compound (VI)); and the step of removing a substituent on an amino group at the 1-position in compound (VI) to obtain a trans-4-(protected amino)piperidin-3-ol represented by formula (VII) (hereinafter abbreviated to compound (VII)) will be described below.

As compound (III-C), the mixture obtained after completion of the reaction may be used as it is, or may be used after the post-treatment. Also, the isolated compound (III-C) or a salt thereof may be used, or the purified compound (III-C) or a salt thereof may be used.

Removal of an allyl type substituent on an amino group at the 4-position of a piperidine ring in compound (III-C) can be carried out by any known method capable of preferentially removing a benzyl type substituent on a nitrogen atom contained in a piperidine ring. Examples of the method include a method of reacting compound (III-C) with palladium on carbon in an alcohol solvent, a method of reacting compound (III-C) with a palladium triphenylphosphine complex, a method of reacting a rhodium chloride triphenylphosphine complex with compound (III-C) and the like, and preferable method is a method of reacting compound (III-C) with palladium on carbon in an alcohol solvent.

Palladium on carbon may be a wet product or a dry product. The content of the palladium atom is preferably from 0.5 to 50% by weight, and more preferably from 5 to 20% by weight. It is also possible to use, as palladium on carbon, commercially available palladium on carbon, and those prepared by any known method. The use amount of the palladium on carbon is usually within a range from 0.1 to 50 g, and preferably from 1 to 20 g, in terms of the palladium atom based on 1 kg of compound (III-C). Palladium supported on carbon is usually zero-valent and when a di- or tetra-valent palladium compound is supported, it is preferably used after reducing to zero valence by a conventional method.

Examples of the alcohol solvent include methanol, ethanol, 1-propanol, 2-propanol, 1-butanol, isobutyl alcohol, tert-butyl alcohol, 1-pentanol, 2-pentanol, isopentyl alcohol, 1-hexanol, 2-hexanol, isohexyl alcohol, 1-heptanol, 2-heptanol, 3-heptanol, isopeptyl alcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-tert-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether and diethylene glycol mono-tert-butyl ether. These alcohol solvents may be used alone, or may be used as a mixture thereof. The alcohol solvent is preferably ethanol. The use amount of the alcohol solvent is preferably from 1 to 50 L, and more preferably from 2 to 15 L, based on 1 kg of compound (III-C).

The reaction of compound (III-C) with palladium on carbon is preferably carried out under an atmosphere of an inert gas such as nitrogen or argon. The present reaction is preferably carried out in the presence of aminoethanol. The use amount of aminoethanol is preferably from 0.5 to 1.5 mol based on 1 mol of compound (III-C). The reaction temperature is preferably within a range from 20 to 130°C, and more preferably from 60 to 90°C. The reaction time varies depending on the reaction temperature, the use amount of a reaction reagent and the like, and is preferably from 1 to 24 hours. The degree of proceeding of the reaction can be confirmed by analysis means such as thin-layer chromatography, gas chromatography and high-performance liquid chromatography.

There is no particular limitation on the order of mixing of reaction reagents, and mixing is preferably carried out by a method of mixing compound (III-C) with aminoethanol in an alcohol solvent and adding palladium on carbon to the obtained mixture, or a method of mixing an alcohol solvent with palladium on carbon and adding compound (III-C) and aminoethanol to the obtained mixture, and more preferably a method of mixing compound (III-C) and aminoethanol in an alcohol solvent and adding palladium on carbon to the obtained mixture.

Compound (V) is contained in the mixture obtained after completion of the reaction, and the mixture obtained after completion of the reaction, which contains the compound, may be subjected to the step of protecting an amino group at the 4-position described hereinafter, or the mixture obtained after completion of the reaction may be subjected to post-treatments such as filtration, extraction and washing with water and then subjected to the step of protecting an amino group at the 4-position. Further, after extracting compound (V) by isolation treatments such as distillation and crystallization, the mixture may be subjected to the step of protecting an amino group at the 4-position. Furthermore, after purifying compound (V) by purification treatments such as recrystallization; extraction and purification; distillation; treatments of absorption to activated carbon, silica, alumina and the like; and chromatography such as silica gel column chromatography, the mixture may be subjected to the step of protecting an amino group at the 4-position Moreover, after extracting compound (V) as a salt of any acid such as hydrochloric acid, benzoic acid or tartaric acid, the mixture may be subjected to the step of protecting an amino group at the 4-position.

Examples of compound (V) include trans-4-amino-1-benzylpiperidin-3-ol, trans-4-amino-1-(1-phenylethyl)piperidin-3-ol, trans-4-amino-1-(1-phenylpropyl)piperidin-3-ol, trans-4-amino-1-(1-phenyl-2-methylpropyl)piperidin-3-ol and trans-4-amino-1-(1-phenylbutyl)piperidin-3-ol. Trans-4-amino-1-benzylpiperidin-3-ol is preferable. When using, as compound (III-C), an optically active substance whose optical activity is attributed to at least one of an asymmetric carbon on a piperidine ring and an asymmetric carbon of a substituent on a nitrogen atom constituting a piperidine ring, the obtained compound (V) is also the optically active substance.

By converting compound (V) to compound (VI), an amino group at the 4-position is protected with an alkoxycarbonyl group. Protection of an amino group at the 4-position in compound (V) is preferably carried out by reacting with an alkyl halocarbonate or a dialkyl carbonate in the presence of a base. Herein, the alkyl halocarbonate is represented by formula (VIII-1): 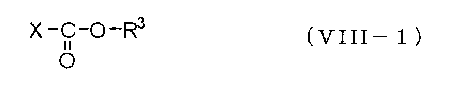 wherein X represents a halogen atom such as a chlorine atom or a bromine atom, and R³ is as defined above, while the dialkyl carbonate is represented by formula (VIII-2): 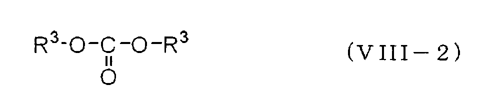 wherein R³ is as defined above.

Examples of the alkyl group having 1 to 12 carbon atoms represented by R³ in formula (VI) include a methyl group, an ethyl group, a propyl group, an isopropyl group, a butyl group, an isobutyl group, a tert-butyl group, a pentyl group, a hexyl group, a heptyl group, an octyl group, a nonyl group, a decyl group, an undecyl group and a dodecyl group, and an ethyl group, an isopropyl group and a tert-butyl group are preferable, and a tert-butyl group is more preferable.

Examples of the base include alkali metal hydroxides such as potassium hydroxide, sodium hydroxide and lithium hydroxide; alkali metal carbonates such as potassium carbonate, sodium carbonate and lithium carbonate; tertiary amines such as triethylamine and diisopropylethylamine; alkali metal alkoxides such as sodium methoxide, sodium ethoxide, sodium tert-butoxide and potassium tert-butoxide; alkali metal hydrides such as sodium hydride and potassium hydride; alkali earth metal hydrides such as calcium hydride; alkyl metal compounds such as n-butyl lithium; and alkali metal amide compounds such as lithium diisopropylamide and lithium hexamethyldisilazide, and tertiary amine is preferable.

Examples of the alkyl halocarbonate include methyl chlorocarbonate, ethyl chlorocarbonate, isopropyl chlorocarbonate and butyl chlorocarbonate. Examples of the dialkyl carbonate include di-tert-butyl carbonate. In order to convert compound (V) to compound (VI) which is a carbamate compound, it is preferable to react a dialkyl carbonate, and more preferable to react a di-tert-butyl carbonate.

The use amount of the base is preferably from 1 to 10 mol, and more preferably from 1 to 3 mol, based on 1 mol of compound (V). The use amount of the alkyl halocarbonate or dialkyl carbonate is preferably from 1 to 5 mol, and more preferably from 1 to 2 mol, based on 1 mol of compound (V). It is also possible to use, as these reagents, commercially available reagents, and those prepared by a known method.

Protection of an amino group is preferably carried out in the presence of a solvent. The solvent may be inert to the reaction, and examples thereof include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, tert-butylcyclohexane and petroleum ether; aromatic solvents such as benzene, toluene, ethylbenzene, isopropylbenzene, tert-butylbenzene, xylene, mesitylene, monochlorobenzene, monofluorobenzene, α,α,α-trifluoromethylbenzene, 1,2-dichlorobenzene, 1,3-dichlorobenzene, 1,2,3-trichlorobenzene and 1,2,4-trichlorobenzene; ether solvents such as tetrahydrofuran, methyltetrahydrofuran, diethyl ether, dipropyl ether, diisopropyl ether, dibutyl ether, dipentyl ether, dihexyl ether, diheptyl ether, dioctyl ether, tert-butyl methyl ether, cyclopentyl methyl ether, 1,2-dimethoxyethane, diethylene glycol dimethyl ether, anisole and diphenylether; aprotic polar solvents such as dimethyl sulfoxide, sulfolane, N,N-dimethylformamide, N,N-dimethylacetamide, N,N-dimethylpropionamide, N-methylpyrrolidone, γ-butyrolactone, dimethyl carbonate, diethyl carbonate, ethylene carbonate, propylene carbonate, 1,3-dimethyl-2-imidazolidinone and 1,3-dimethyl-3,4,5,6-tetrahydro-2(1H)-pyridinone; nitrile solvents such as acetonitrile and propionitrile; water; and mixtures thereof. Among these solvents, ether solvents are preferable, and tetrahydrofuran is more preferable. The use amount of the solvent is preferably from 1 to 50 L, and more preferably from 2 to 15 L, based on 1 kg of compound (V).

The reaction temperature is preferably within a range from -30°C to 70°C, and more preferably from 0°C to 50°C. The reaction time varies depending on the reaction temperature, the use amount of a reaction reagent and the like, and is preferably from 1 to 20 hours. The degree of proceeding of the reaction can be confirmed by analysis means such as thin-layer chromatography, gas chromatography and high-performance liquid chromatography.

There is no particular limitation on the order of mixing of reaction reagents. However, mixing is preferably carried out in such an order that a base is added in a mixture of compound (V) and a solvent and then an alkyl halocarbonate or a dialkyl carbonate is added.

Compound (VI) is contained in the mixture obtained after completion of the reaction, and the mixture obtained after completion of the reaction, which contains the compound, may be subjected to the step of removing a substituent on an amino group at the 1-position described hereinafter. For example, the mixture obtained after completion of the reaction may be subjected to post-treatments such as filtration, extraction and washing with water and then subjected to the method of removing a substituent on an amino group at the 1-position. Further, after extracting compound (VI) by isolation treatments such as distillation and crystallization, the mixture may be subjected to the step of removing a substituent on an amino group at the 1-position. Furthermore, after purifying compound (VI) by purification treatments such as recrystallization; extraction and purification; distillation; treatments of absorption to activated carbon, silica, alumina and the like; and chromatography such as silica gel column chromatography, the mixture may be subjected to the step of removing a substituent on an amino group at the 1-position. Further, after extracting compound (VI) as a salt of any acid such as hydrochloric acid, benzoic acid or tartaric acid, the mixture may be subjected to the step of removing a substituent on an amino group at the 1-position.

Examples of compound (VI) include methyl 1-benzyl-trans-3-hydroxypiperidin-4-ylcarbamate, methyl 1-(1-phenylethyl)-trans-3-hydroxypiperidin-4-ylcarbamate, methyl 1-(1-phenylpropyl)-trans-3-hydroxypiperidin-4-ylcarbamate, methyl 1-(1-phenyl-2-methylpropyl)-trans-3-hydroxypiperidin-4-ylcarbamate, ethyl 1-benzyl-trans-3-hydroxypiperidin-4-ylcarbamate, ethyl 1-(1-phenylethyl)-trans-3-hydroxypiperidin-4-ylcarbamate, ethyl 1-(1-phenylpropyl)-trans-3-hydroxypiperidin-4-ylcarbamate, ethyl 1-(1-phenyl-2-methylpropyl)-trans-3-hydroxypiperidin-4-ylcarbamate, isopropyl 1-benzyl-trans-3-hydroxypiperidin-4-ylcarbamate, isopropyl 1-(1-phenylethyl)-trans-3-hydroxypiperidin-4-ylcarbamate, isopropyl 1-(1-phenylpropyl)-trans-3-hydroxypiperidin-4-ylcarbamate, isopropyl 1-(1-phenyl-2-methylpropyl)-trans-3-hydroxypiperidin-4-ylcarbamate, tert-butyl 1-benzyl-trans-3-hydroxypiperidin-4-ylcarbamate, tert-butyl 1-(1-phenylethyl)-trans-3-hydroxypiperidin-4-ylcarbamate, tert-butyl 1-(1-phenylpropyl)-trans-3-hydroxypiperidin-4-ylcarbamate and tert-butyl 1-(1-phenyl-2-methylpropyl)-trans-3-hydroxypiperidin-4-ylcarbamate. Tert-butyl 1-benzyl-trans-3-hydroxypiperidin-4-ylcarbamate is preferable. When at least one of the compound (V), alkyl halocarbonate and dialkyl carbonate is an optically active substance, the obtained compound (III-B) is also the optically active substance.

By converting compound (VI) to compound (VII), a substituent on an amino group at the 1-position is removed. Removal of a substituent on an amino group at the 1-position in compound (VI) is preferably carried out under the condition inert to an amino group protected with an alkoxycarbonyl group. Examples of the method for removal include a method of reacting compound (VI) with hydrogen in the presence of palladium on carbon, a method of reacting compound (VI) with hydrogen in the presence of palladium hydroxide, a method of reacting compound (VI) with sodium in liquid ammonia, and a method of reacting compound (VI) with hydrogen in the presence of palladium on carbon is preferable.

Palladium on carbon may be a wet product or a dry product. The content of the palladium atom is preferably from 0.5 to 50% by weight, and more preferably from 5 to 20% by weight. It is also possible to use, as the palladium on carbon, commercially available palladium on carbon, and those prepared by any known method. The use amount of the palladium on carbon is usually within a range from 0.1 to 50 g, and preferably from 1 to 20 g, in terms of the palladium atom based on 1 kg of compound (VI). Palladium supported on carbon is preferably zero-valent and when a di- or tetra-valent palladium compound is supported, it is preferably used after reducing to zero valence by a conventional method.

It is also possible to use, as hydrogen, a commercially available hydrogen gas, and those generated by any known method. The hydrogen pressure during the reaction is preferably from 0.1 to 5 MPa, and more preferably from 0.1 to 1 MPa. It is also possible to use as a mixed gas with an inert gas such as nitrogen or argon. In that case, the hydrogen partial pressure during the reaction is the same as the above hydrogen pressure.

The reaction of compound (VI) with hydrogen is preferably carried out in the presence of a solvent. The solvent may not inhibit the reaction, and examples thereof include aliphatic hydrocarbon solvents such as pentane, hexane, isohexane, heptane, isoheptane, octane, isooctane, nonane, isononane, decane, isodecane, undecane, dodecane, cyclopentane, cyclohexane, methylcyclohexane, tert-butylcyclohexane and petroleum ether; ether solvents such as tetrahydrofuran, methyltetrahydrofuran, diethyl ether, dipropyl ether, diisopropyl ether, dibutyl ether, dipentyl ether, dihexyl ether, diheptyl ether, dioctyl ether, tert-butyl methyl ether, cyclopentyl methyl ether, 1,2-dimethoxyethane and diethylene glycol dimethyl ether; alcohol solvents such as methanol, ethanol, 1-propanol, 2-propanol, 1-butanol, isobutyl alcohol, tert-butyl alcohol, 1-pentanol, 2-pentanol, isopentyl alcohol, 1-hexanol, 2-hexanol, isohexyl alcohol, 1-heptanol, 2-heptanol, 3-heptanol, isopeptylalcohol, ethylene glycol monomethyl ether, ethylene glycol monoethyl ether, ethylene glycol monopropyl ether, ethylene glycol monoisopropyl ether, ethylene glycol monobutyl ether, ethylene glycol monoisobutyl ether, ethylene glycol mono-tert-butyl ether, diethylene glycol monomethyl ether, diethylene glycol monoethyl ether, diethylene glycol monopropyl ether, diethylene glycol monoisopropyl ether, diethylene glycol monobutyl ether, diethylene glycol monoisobutyl ether and diethylene glycol mono-tert-butyl ether; ester solvents such as ethyl acetate, propyl acetate, isopropyl acetate, butyl acetate, isobutyl acetate, tert-butyl acetate, amyl acetate and isoamyl acetate; aprotic polar solvents such as dimethyl sulfoxide, sulfolane, N,N-dimethylformamide, N,N-dimethylacetamide, N,N-dimethylpropionamide, N-methylpyrrolidone, γ-butyrolactone, dimethyl carbonate, diethyl carbonate, ethylene carbonate, propylene carbonate, 1,3-dimethyl-2-imidazolidinone and 1,3-dimethyl-3,4,5,6-tetrahydro-2(1H)-pyridinone; water; and mixtures thereof. Among these solvents, alcohol solvents are preferable, and ethanol is more preferable. The use amount of the solvent is preferably from 1 to 50 L, and more preferably from 2 to 15 L, based on 1 kg of compound (VI).

The reaction temperature is preferably within a range from 0 to 100°C, and more preferably from 20 to 70°C. The reaction time varies depending on the reaction temperature, the use amount of a reaction reagent, the hydrogen pressure and the like, and is preferably from 1 to 24 hours. The degree of proceeding of the reaction can be confirmed by analysis means such as thin-layer chromatography, gas chromatography and high-performance liquid chromatography.

There is no particular limitation on the order of mixing of reaction reagents. For example, mixing can be carried out by a method of mixing compound (VI) or a solution thereof with palladium on carbon and adding hydrogen to the obtained mixture, a method of adding compound (VI) to palladium on carbon under a hydrogen atmosphere and the like. A method of mixing a solution of compound (VI) with palladium on carbon and adding hydrogen to the obtained mixture is preferable.

Compound (VII) is contained in the mixture obtained after completion of the reaction and, when the mixture obtained after completion of the reaction, which contains compound (VII) is subjected to post-treatments such as filtration, extraction and washing with water and then subjected to isolation treatments such as distillation and crystallization, compound (VII) can be extracted. At this time, compound (VII) may be extracted as a salt of any acid such as hydrochloric acid, benzoic acid or tartaric acid. The extracted compound (VII) or a salt thereof can be prepared by purification treatments such as recrystallization; extraction and purification; distillation; treatments of absorption to activated carbon, silica, alumina and the like; and chromatography such as silica gel column chromatography.

Examples of compound (VII) include methyl trans-3-hydroxypiperidin-4-ylcarbamate, ethyl trans-3-hydroxypiperidin-4-ylcarbamate, isopropyl trans-3-hydroxypiperidin-4-ylcarbamate and tert-butyl trans-3-hydroxypiperidin-4-ylcarbamate. Tert-butyl trans-3-hydroxypiperidin-4-ylcarbamate is preferable. When using, as the compound (VI), an optically active substance whose optical activity is attributed to an asymmetric carbon atom on a piperidine ring and an asymmetric carbon atom contained in an alkoxycarbonyl group, the obtained compound (VII) is also the optically active substance.

In the reaction of compound (I) with compound (II), when using, as compound (II), a compound represented by formula (II-D): 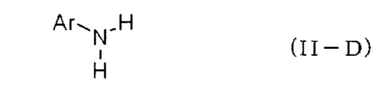 wherein Ar is as defined above (hereinafter abbreviated to a compound (II-D)), a compound represented by formula (III-D) : 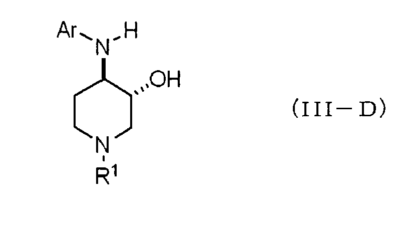 wherein R¹ and Ar are as defined above, can be obtained.

In formula (II-D), Ar represents a phenyl group which may be substituted, examples of alkyl group having 1 to 12 carbon atoms as the substituent include a methyl group, an ethyl group, a propyl group, an isopropyl group, a n-butyl group, an isobutyl group, a tert-butyl group, a pentyl group, a hexyl group, a heptyl group, an octyl group, a nonyl group, a decyl group, an undecyl group and a dodecyl group; examples of the alkoxy group having 1 to 12 carbon atoms include a methoxy group, an ethoxy group, a propoxy group and a butoxy group; examples of the halogen atom include a fluorine group, a chlorine group, a bromine group and an iodine group; examples of the protected amino group include an acetylamino group, a benzoylamino group, a methoxycarbonylamino group and an ethoxycarbonylamino group; and examples of the protected hydroxyl group include an acetoxy group, a benzoyloxy group, a benzyloxy group, a methoxymethoxy group and a tert-butyldimethylsilyloxy group. Examples of Ar include a 2-methylphenyl group, a 3-methylphenyl group, a 4-methylphenyl group, a 2, 4-dimethylphenyl group, a 4-ethylphenyl group, a 4-propylphenyl group, a 2-methoxyphenyl group, a 3-methoxyphenyl group, a 4-methoxyphenyl group, a 2-bromophenyl group, a 3-bromophenyl group, a 4-bromophenyl group, a 4-(acetylamino)phenyl group and a 4-(methoxycarbonylamino)phenyl group.

Examples of compound (II-D) include aniline, o-toluidine, m-toluidine, p-toluidine, 2,4-xylidine, 4-ethylaniline, 4-propylaniline, o-anisidine, m-anisidine, p-anisidine, 3-bromoaniline, 4-(acetylamino)aniline and methyl(4-aminophenyl) carbamate.

Examples of compound (III-D) include trans-1-benzyl-4-(phenylamino)piperidin-3-ol, trans-1-benzyl-4-(o-tolylamino)piperidin-3-ol, trans-1-benzyl-4-(m-tolylamino)piperidin-3-ol, trans-1-benzyl-4-(p-tolylamino)piperidin-3-ol, trans-1-benzyl-4-[(2-methoxyphenyl)amino]piperidin-3-ol, trans-1-benzyl-4-[(2,4-dimethylphenyl)amino]piperidin-3-ol, trans-1-benzyl-4-[(4-ethylphenyl)amino]piperidin-3-ol, trans-1-benzyl-4-[(3-bromophenyl)amino]piperidin-3-ol, trans-4-{(4-acetylamino)phenyl}amino}-1-benzylpiperidin-3-ol, methyl 4-[(1-benzyl-3-hydropiperidin-4-yl)amino]phenylcarbamate, trans-4-(phenylamino)-1-(1-phenylethyl)piperidin-3-ol, trans-4-(o-tolylamino)-1-(1-phenylethyl)piperidin-3-ol, trans-4-(m-tolylamino)-1-(1-phenylethyl)piperidin-3-ol, trans-4-(p-tolylamino)-1-(1-phenylethyl)piperidin-3-ol, trans-4-[(2-methoxyphenyl)amino]-1-(1-phenylethyl)piperidin-3-ol, trans-4-[(2,4-dimethylphenyl)amino]-1-(1-phenylethyl)piperidin-3-ol, trans-4-[(4-ethylphenyl)amino]-1-(1-phenylethyl)piperidin-3-ol, trans-4-[(3-bromophenyl)amino]-1-(1-phenylethyl)piperidin-3-ol, trans-4-{(4-acetylamino)phenyl}amino}-1-(1-phenylethyl)piperidin-3-ol and methyl 4-[3-hydro-1-(1-phenylethyl)piperidin-4-yl]amino]phenylcarbamate. When a racemic form is used as compound (I), the obtained compound (III-B) is also the racemic form and, when an optically active substance is used as compound (I), the obtained compound (III-B) is also the optically active substance.

### EXAMPLES

The present invention will be described in more detail below, but the present invention is not limited to these Examples.

### Reference Example 1: Production of 1-benzyl-1,2,3,6-tetrahydropyridine

After mixing 10 g (126 mmol) of pyridine with 20 mL of acetonitrile, 16.2 g (126 mmol) of benzyl bromide was added dropwise thereto at room temperature over 1 hour. After completion of the dropwise addition, the obtained mixture was adjusted to a temperature of 70 to 72°C and stirred at the same temperature for 3 hours. After completion of the reaction, the reaction mixture was cooled to about room temperature and the solvent was distilled off from the reaction mixture, followed by mixing with 150 mL of ethanol. To the obtained mixture, 9.53 g (252 mmol) of sodium borohydride was added in four portions over 1 hour. The obtained mixture was stirred at room temperature for 12 hours. After completion of the reaction, the reaction mixture was adjusted to 15°C and 100 mL of water was added dropwise thereto. After mixing, 13.7 g of 35% by weight of hydrochloric acid was added, followed by stirring for 1 hour. Thereafter, an aqueous 25% by weight of sodium hydroxide solution was added until the aqueous layer of the reaction mixture exhibits pH 9, and the solution was extracted with 200 mL of toluene. The obtained organic layer was washed with 50 mL of saturated saline. The obtained organic layer was dried over sodium sulfate and then the solvent was distilled off under reduced pressure. The obtained concentrated residue was distilled under the condition of 102-107°C /0.6 kPa to obtain 8.6 g of 1-benzyl-1,2,3,6-tetrahydropyridine. Yield: 40%.

### Reference Example 2: Production of 3-benzyl-7-oxa-3-azabicyclo[4.1.0]heptane

After mixing 7.77 g (44.8 mmol) of 1-benzyl-1,2,3,6-tetrahydropyridine obtained in Reference Example 1 with 50 mL of water, 5.11 g (44.8 mmol) of trifluoroacetic acid was added dropwise thereto at room temperature over 10 minutes. To the obtained mixture, 7.18 g (53.8 mmol) of N-chlorosuccinimide was added little by little at room temperature over 1 hour. The reaction mixture was adjusted to 45°C and stirred at the same temperature for 2.5 hours and, after returning to room temperature, the mixture was stirred overnight. Even after stirring overnight at room temperature, the reaction was not completed. Therefore, the reaction mixture was adjusted again to 45°C and stirred at the same temperature for 7 hours. After completion of the reaction, the reaction mixture was adjusted to 12°C and 25 mL of toluene was added thereto, and further 27.3 g of an aqueous 48% by weight of sodium hydroxide solution was added dropwise over 30 minutes. The obtained mixture was adjusted to 40°C and stirred at the same temperature for 5.5 hours. After stirring was terminated, liquid separation was carried out and the aqueous layer was extracted again with 25 mL of toluene. The organic layer were combined and washed with 25 mL of saturated saline. The obtained organic layer was dried over sodium sulfate and then the solvent was distilled off under reduced pressure to obtain 7.68 g of 3-benzyl-7-oxa-3-azabicyclo[4.1.0]heptane. Yield: 90%.

### Example 1: Production of (3RS,4RS)-1-benzyl-4-benzylaminopiperidin-3-ol

After mixing 0.50 g (2.6 mmol) of 3-benzyl-7-oxa-3-azabicyclo[4.1.0]heptane obtained in Reference Example 2 with 4.5 mL of acetonitrile, 0.43 mL (4.0 mmol) of benzylamine and 1.33 g (12.5 mmol) of lithium perchlorate were added thereto, and then the obtained mixture was reacted at room temperature for 3 hours. After completion of the reaction, 5 mL of water was added to the obtained mixture, then the solution was extracted with 10 mL of toluene and, further the obtained aqueous layer was extracted again with 5 mL of toluene. The obtained organic layers were combined and dried over sodium sulfate, and then the solvent was distilled off under reduced pressure to obtain 0.74 g of (3RS,4RS)-1-benzyl-4-benzylaminopiperidin-3-ol as a crystal. Yield: 95%. ¹H-NMR of the obtained crystal was measured. As a result, a peak attributed to (3RS,4RS)-1-benzyl-3-benzylaminopiperidin-4-ol was not observed.

### Examples 2 to 7, Comparative Example 1

In the same manner as in Example 1, except that the conditions shown in Table 1 were changed in Example 1, the reaction was carried out.

The results are shown in Table 1.

**Table 1**

| Examples | Benzylamine (molar-fold)* | Lithium salt (molar-fold)* | Reaction temperature, Reaction time | Yield | Position isomer ratio** |
|---|---|---|---|---|---|
| 1 | 1.5 | LiClO₄ (4.7) | Room temperature, 3 hours | 95% | > 20:1 |
| 2 | 1.5 | LiCl (5.0) | Room temperature, 18.5 hours | 95% | > 20:1 |
| 3 | 1.2 | LiCl (1.0) | Room temperature, 14 hours | 92% | > 20:1 |
| 4 | 1.2 | LiCl (0.7) | Room temperature, 24 hours | 95% | > 20:1 |
| 5 | 1.2 | LiCl (0.5) | Room temperature, 24 hours | 91% | 20:1 |
| 6 | 1.2 | LiCl (0.2) | Room temperature, 48 hours | 64% | 10:1 |
| 7 | 1.0 | LiCl (0.2) | 40°C, 45 hours | 88% | < 6:1 |
| Comparative 1,5 Example 1 | | None | 70°C, 18 hours | < 30% | 1:6 |

| | | | | | |
|---|---|---|---|---|---|
| * : In any case, molar-fold is based on 1 mol of 3-benzyl-7-oxa-3-aza-bicyclo[4.1.0]heptane ** : A position isomer ratio is a production ratio of the following two compounds. | | | | | |

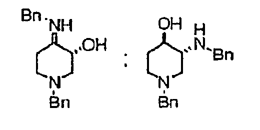

### Examples 8 to 12

In the same manner as in Example 1, except that the conditions shown in Table 2 were changed in Example 1, the reaction was carried out. The results are shown in Table 3.

**Table 2**

| Examples | Amina (molar-fold)* | Lithium salt (molar-fold)* | Reaction temperature, Reaction time |
|---|---|---|---|
| 8 | Dibenzylamine (1.5) | LiClO₄ (5.0) | Room temperature, 2 hours → 45°C, 7 hours |
| 9 | (R)-1-phenylethylamine (1.5) | LiClO₄ (2.0) | Room temperature, 5.5 hours |
| 10 | Allylamine (1.5) | LiCl (1.1) | Room temperature, 21 hours → 40°C, 1.5 hours |
| 11 | Aniline (1.0) | LiCl (2.0) | 50 to 60°C, 24 hours |
| 12 | o-anisidine (1.0) | LiCl (2.0) | 60°C, 18 hours |

| | | | |
|---|---|---|---|
| * : In any case, molar-fold is based on 1 mol of 3-benzyl-7-oxa-3-aza-bicyclo[4.1.0]heptane | | | |

**Table 3**

| Examples | Products | Yield |
|---|---|---|
| 8 | 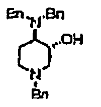 | 89% |
| 9 | 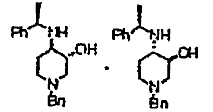 | 92% (diastereomer ratio = 1:1) |
| 10 | 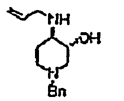 | 75% |
| 11 | 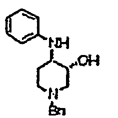 | 56% |
| 12 | 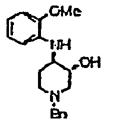 | 64% |

### Example 13: Production of (3RS,4RS)-1-benzyl-4-(pyrrolidin-1-yl)piperidin-3-ol

After mixing 0.47 g (2.5 mmol) of 3-benzyl-7-oxa-3-azabicyclo[4.1.0]heptane obtained in Reference Example 2 with 5 mL of acetonitrile, 0.24 mL (2.7 mmol) of pyrrolidine and 70 mg (1.7 mmol) of lithium chloride were added thereto, and then the obtained mixture was reacted at room temperature for 24 hours. After completion of the reaction, the reaction solution was concentrated to dryness as it is to obtain 0.74 g of a mixture of (3RS, 4RS)-1-benzyl-4-(pyrrolidin-1-yl)piperidin-3-ol and acetonitrile.
¹H-NMR spectrum of the mixture was measured. As a result, 86% of (3RS, 4RS)-1-benzyl-4-(pyrrolidin-1-yl)piperidin-3-ol was contained. Yield: 98%.

### Example 14: Production of (3RS, 4RS)-4-amino-3-hydroxypiperidine

After mixing 1.01 g (3.4 mmol) of (3RS, 4RS)-1-benzyl-4-benzylaminopiperidin-3-ol obtained in the same manner as in Example 1 and 10 mL of ethanol in an autoclave reactor, the atmosphere inside the system was replaced by a nitrogen atmosphere. After adding 0.10 g of 10% by weight of palladium on carbon (55% by weight a hydrate product, PE type, manufactured by N.E. CHEMCAT CORPORATION, Lot. 217-076880) thereto, the atmosphere inside the system was replaced by hydrogen, followed by stirring under a hydrogen pressure of 0.4 MPa at 50°C for 6 hours. After completion of the reaction, the catalyst was removed by filtration and the obtained filtrate was concentrated to quantitatively obtain 0.42 g of (3RS, 4RS)-4-amino-3-hydroxypiperidine.
¹H-NMR of the obtained compound was measured. As a result, a peak assigned to (3RS, 4RS)-3-aminopiperidin-4-ol was not recognized.

### Example 15: Production of (3RS, 4RS)-4-amino-1-benzylpiperidin-3-ol

After mixing 0.14 g (0.55 mmol) of (3RS,4RS)-1-benzyl-4-allylaminopiperidin-3-ol obtained in Example 10 with 2 mL of ethanol in a flask, the atmosphere inside the system was replaced by a nitrogen atmosphere. After adding 0.04 mL (0.66 mmol) of aminoethanol and 0.014 g of 10% by weight of palladium on carbon (55% by weight a hydrate product, PE type, manufactured by N.E. CHEMCAT CORPORATION, Lot. 217-076880) thereto, contents were stirred for 5 hours while heating the flask in an oil bath at 70 to 75°C and the oil was heated within a range from 80 to 85°C, followed by stirring for 3 hours. The degree of proceeding of the reaction was confirmed by thin-layer chromatography. However, since (3RS,4RS)-1-benzyl-4-allylaminopiperidin-3-ol remained, the mixture was cooled to about room temperature and 0.014 g of 10% by weight of palladium on carbon was added, and then contents were stirred for 2 hours while heating the flask again in a hot water bath at 80 to 85°C. After completion of the reaction, the catalyst was removed by filtration and the obtained filtrate was concentrated. To the obtained concentrated residue, 2 mL of water and 1 mL of aqueous 1M sulfuric acid were added and, after stirring at room temperature for 1 hour, the solution was washed with 4 mL of ethyl acetate. Furthermore, the obtained organic layer was back-extracted with 1.5 mL of water and 1.5 mL of 1M sulfuric acid solution. The obtained aqueous layers were combined and the pH was adjusted to 9 by adding dropwise 50% by weight of potassium hydroxide solution, and then the solution was extracted three times with 10 mL of n-butanol. The obtained organic layers were combined and the obtained mixture was partially concentrated, and then ethyl acetate was added to the concentrated residue. As a result, a salt was precipitated and therefore the salt was removed by filtration. The obtained filtrate was concentrated to obtain 0.18 g of a mixture containing (3RS, 4RS)-4-amino-1-benzylpiperidin-3-ol.

### Example 16: Production of tert-butyl (3RS, 4RS)-1-benzyl-3-hydroxypiperidin-4-ylcarbamate

After mixing 0.18 g of a mixture containing (3RS,4RS)-4-amino-1-benzylpiperidin-3-ol obtained in Example 15 with 2 mL of tetrahydrofuran, 0.09 mL (0.65 mmol) of triethylamine and 0.14 g (0.65 mmol) of di-tert-butyl dicarbonate were added thereto and the obtained mixture was stirred at room temperature for 16 hours. After completion of the reaction, 2 mL of water was added to the reaction mixture and then the solution was extracted with 5 mL of toluene, and also the obtained aqueous layer was extracted again with 2 mL of toluene. The obtained organic layers were combined, washed with saturated saline, dried over anhydrous sodium sulfate and then concentrated. The obtained residue was purified by silica gel column chromatography (n-heptane/ethyl acetate = 2/1 to only ethyl acetate) to obtain 0.12 g of tert-butyl (3RS, 4RS)-1-benzyl-3-hydroxypiperidin-4-ylcarbamate. A total yield from Example 10 was 70% (based on (3RS, 4RS)-1-benzyl-4-benzylaminopiperidin-3-ol).

### Example 17: Production of tert-butyl (3RS, 4RS)-3-hydroxypiperidin-4-ylcarbamate

After mixing 0.12 g (0.39 mmol) of tert-butyl (3RS,4RS)-1-benzyl-3-hydroxypiperidin-4-ylcarbamate obtained in Example 16 and 4 mL of ethanol in an autoclave reactor, the atmosphere inside the system was replaced by a nitrogen atmosphere. After adding 0.017 g of 10% by weight of palladium on carbon (55% by weight a hydrate product, PE type, manufactured by N.E. CHEMCAT CORPORATION, Lot. 217-076880) thereto, the atmosphere inside the system was replaced by hydrogen, followed by stirring under a hydrogen pressure of 0.5 MPa at 50°C for 4 hours. After completion of the reaction, the catalyst was removed by filtration and the obtained filtrate was concentrated to obtain 0.072 g of tert-butyl (3RS, 4RS)-3-hydroxypiperidin-4-ylcarbamate.
Yield: 85%

### Comparative Example 2

In the same manner as in Example 1, except that the same molar amount of ethyl 7-oxa-3-azabicyclo[4.1.0]heptane-3-carboxylate was used in place of 3-benzyl-7-oxa-3-azabicyclo[4.1.0]heptane in Example 1, the reaction was carried out. After reacting at room temperature for 18 hours, analysis was carried out by thin-layer chromatography. As a result, since the raw materials remained, the reaction mixture was heated in a hot water bath at 40°C and further reacted at room temperature for 5 hours, and then subjected to a post-treatment by the method described in Example 1. The obtained concentrated residue was purified by silica gel column chromatography (n-heptane/ethyl acetate = 2:1 to only ethyl acetate) thereby separating into the product and the raw material. As a result, 30% of the raw material was recovered. ¹H-NMR of the product was measured. As a result, a production ratio of ethyl trans-4-benzylamino-3-hydroxypiperidine-1-carboxylate to ethyl trans-3-benzylamino-4-hydroxypiperidine-1-carboxylate was about 2:1. The total yield of these two position isomers was 51%.

### INDUSTRIAL APPLICABILITY

According to the present invention, 1-substituted-trans-4-(substituted amino)piperidin-3-ol represented by formula (III-1), which has high position selectivity in introduction of a substituted amino group and is useful as a pharmaceutical intermediate, is selectively obtained. Since 1-substituted-trans-4-(substituted amino)piperidin-3-ol can be converted to a trans-4-aminopiperidin-3-ol compound and this trans-4-aminopiperidin-3-ol compound is useful as various chemical products such as a pharmaceutical intermediate (see, for example, International Publication No. WO 2007/039462 or the like), the present invention can be utilized as a method for producing such a compound.

## Claims

1. A method for producing a 1-substituted-trans-4-(substituted amino)piperidin-3-ol represented by formula (III-1): 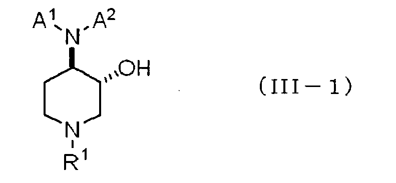 wherein R¹, A¹ and A² are as defined below, which comprises a step of reacting a 1-substituted-3,4-epoxypiperidine represented by formula (I): 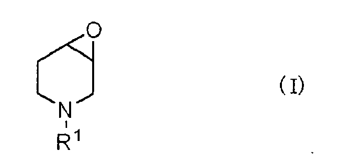 wherein R¹ represents an aromatic carbocyclic group, an alkyl group having 1 to 12 carbon atoms which may be substituted with one or more aromatic carbocyclic groups,
an alkenyl group having 2 to 14 carbon atoms which may be substituted with one or more aromatic carbocyclic groups,
or an alkynyl group having 2 to 12 carbon atoms which may be substituted with one or more aromatic carbocyclic groups, in which each aromatic carbocyclic group may be substituted with one or more substituents selected from the group consisting of an alkyl group having 1 to 12 carbon atoms,
an alkoxy group having 1 to 12 carbon atoms, a halogen atom, a protected amino group and a protected hydroxyl group, with an amine compound represented by formula (II) 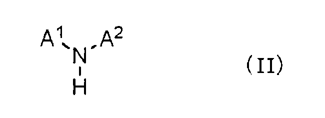 wherein A¹ represents an aromatic carbocyclic group, an alkyl group having 1 to 12 carbon atoms which may be substituted with one or more aromatic carbocyclic groups, an alkenyl group having 2 to 14 carbon atoms which may be substituted with one or more aromatic carbocyclic groups, or an alkynyl group having 2 to 12 carbon atoms which may be substituted with one or more aromatic carbocyclic groups, A² represents an alkyl group having 1 to 12 carbon atoms which may be substituted with one or more aromatic carbocyclic groups, an alkenyl group having 2 to 14 carbon atoms which may be substituted with one or more aromatic carbocyclic groups, an alkynyl group having 2 to 12 carbon atoms which may be substituted with one or more aromatic carbocyclic groups, or a hydrogen atom, or A¹ and A² represent a polymethylene group having 2 to 7 carbon atoms formed by combining with each other, in which each aromatic carbocyclic group may be substituted with one or more substituents selected from the group consisting of an alkyl group having 1 to 12 carbon atoms, an alkoxy group having 1 to 12 carbon atoms, a halogen atom, a protected amino group and a protected hydroxyl group, in the presence of an inorganic lithium salt.

2. The method according to claim 1, wherein the inorganic lithium salt is lithium halide or lithium perhalogenate.

3. The method according to claim 1, wherein the inorganic lithium salt is lithium chloride or lithium perchlorate.

4. The method according to any one of claims 1 to 3, wherein the 1-substituted-3,4-epoxypiperidine represented by formula (I) is a compound represented by formula (I-A) 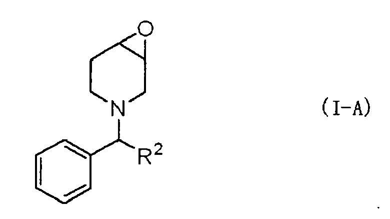 wherein R² represents an aralkyl group having 7 to 17 carbon atoms, an alkyl group having 1 to 11 carbon atoms, a phenyl group or a hydrogen atom, the amine compound represented by formula (II) is a compound represented by formula (II-A): 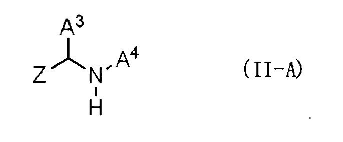 wherein A³ represents a hydrogen atom or an alkyl group having 1 to 11 carbon atom, A⁴ represents a hydrogen atom, a benzyl group or an allyl group, and Z represents a phenyl group or a vinyl group, and the 1-substituted-trans-4-substituted aminopiperidin-3-ol represented by formula (III-1) is a compound represented by formula (III-A): 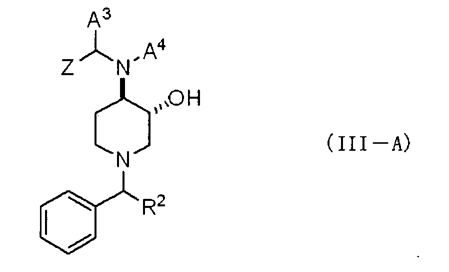 wherein R², A³, A⁴ and Z are as defined above.

5. The method according to any one of claims 1 to 3, wherein the 1-substituted-3,4-epoxypiperidine represented by formula (I) is a compound represented by formula (I-A): 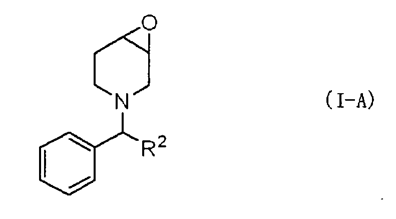 wherein R² represents an aralkyl group having 7 to 17 carbon atoms, an alkyl group having 1 to 11 carbon atoms, a phenyl group or a hydrogen atom, the amine compound represented by formula (II) is a compound represented by formula (II-B): 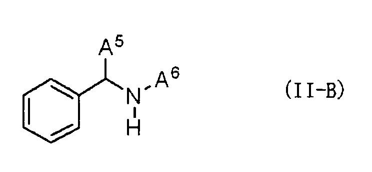 wherein A⁵ represents a hydrogen atom or an alkyl group having 1 to 11 carbon atom, and A⁶ represents a hydrogen atom or a benzyl group, and the 1-substituted-trans-4-substituted aminopiperidin-3-ol represented by formula (III-1) is a compound represented by formula (III-B): 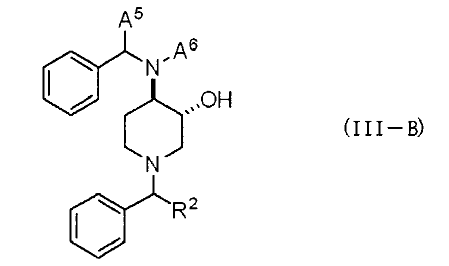 wherein A⁵, A⁶ and R² are respectively as defined above.

6. The method according to claim 5, wherein R² in formulas (I-A) and (III-B) is a hydrogen atom, and both A⁵ and A⁶ in formulas (II-B) and (III-B) are hydrogen atoms.

7. A method for producing trans-4-aminopiperidin-3-ol represented by formula (IV): 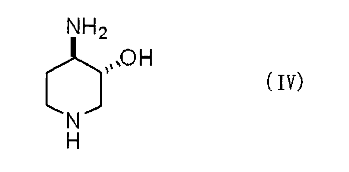 which comprises a step of reacting a 1-substituted-3,4-epoxypiperidine represented by formula (I-A): 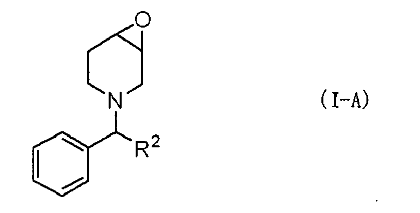 wherein R² represents an aralkyl group having 7 to 17 carbon atoms, an alkyl group having 1 to 11 carbon atoms, a phenyl group or a hydrogen atom,
with an amine compound represented by formula (II-B): 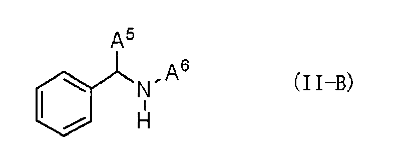 wherein A⁵ represents a hydrogen atom or an alkyl group having 1 to 11 carbon atom, and A⁶ represents a hydrogen atom or a benzyl group, in the presence of an inorganic lithium salt to obtain a 1-substituted-trans-4-(substituted amino)piperidin-3-ol represented by formula (III-B): 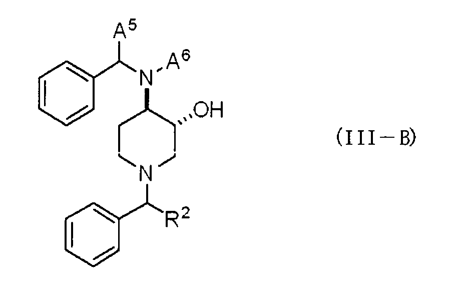 wherein A⁵, A⁶ and R² are respectively as defined above; and a step of reducing the 1-substituted-trans-4-(substituted amino)piperidin-3-ol represented by formula (III-B).

8. The producing method according to any one of claims 1 to 3, wherein the 1-substituted-3,4-epoxypiperidine represented by formula (I) is a compound represented by formula (I-A): 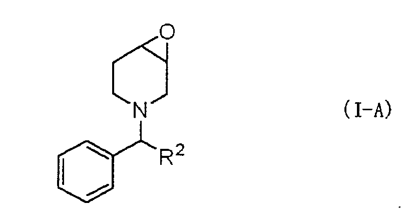 wherein R² represents an aralkyl group having 7 to 17 carbon atoms, an alkyl group having 1 to 11 carbon atoms, a phenyl group or a hydrogen atom, the amine compound represented by formula (II) is a compound represented by formula (II-C): 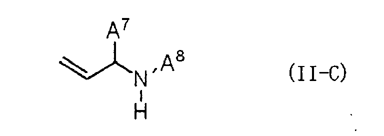 wherein A⁷ represents a hydrogen atom or an alkyl group having 1 to 11 carbon atom, and A⁸ represents a hydrogen atom or an allyl group, and the 1-substituted-trans-4-substituted aminopiperidin-3-ol represented by formula (III-1) is a compound represented by formula (III-C): 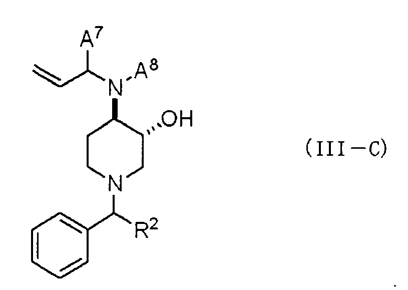 wherein A⁷, A⁸ and R² are respectively as defined above.

9. The producing method according to claim 8,
wherein R² in formulas (I-A) and (III-C) is a hydrogen atom, and both A⁷ and A⁸ in formulas (II-C) and (III-C) are hydrogen atoms.

10. A method for producing trans-4-(protected amino)piperidin-3-ol represented by formula (VII): 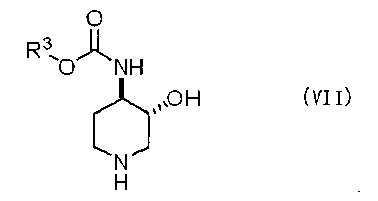 wherein R³ is as defined below, which comprises a step of reacting a 1-substituted-3,4-epoxypiperidine represented by formula (I-A): 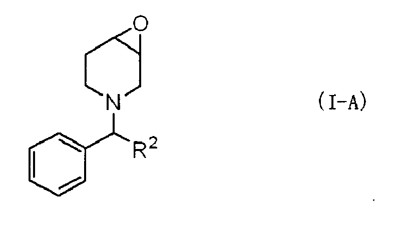 wherein R² represents an aralkyl group having 7 to 17 carbon atoms, an alkyl group having 1 to 11 carbon atoms, a phenyl group or a hydrogen atom,
with an amine compound represented by formula (II-C): 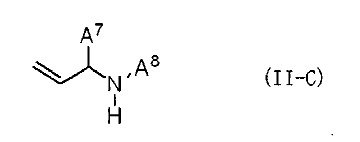 wherein A⁷ represents a hydrogen atom or an alkyl group having 1 to 11 carbon atom, and A⁸ represents a hydrogen atom or an allyl group, in the presence of an inorganic lithium salt to obtain a 1-substituted-trans-4-(substituted amino)piperidin-3-ol represented by formula (III-C): 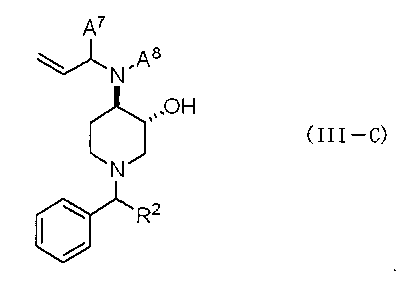 wherein A⁷, A⁸ and R² are as defined above;
a step of removing a substituent on an amino group at the 4-position in the 1-substituted-trans-4-(substituted amino)piperidin-3-ol represented by formula (III-C) to obtain a 1-substituted-trans-4-aminopiperidin-3-ol represented by formula (V): 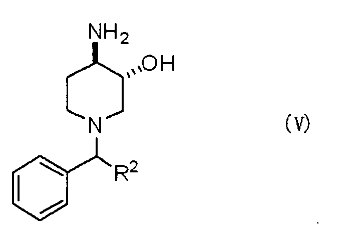 wherein R² is as defined above;
a step of protecting an amino group at the 4-position in the 1-substituted-trans-4-aminopiperidin-3-ol represented by formula (V) to obtain a 1-substituted-trans-4-(protected amino)piperidin-3-ol represented by formula (VI): 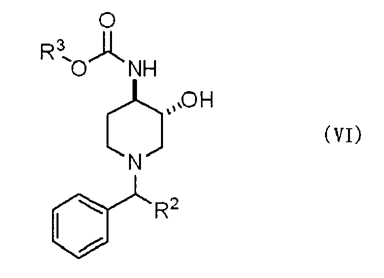 wherein R² is as defined above, and R³ represents an alkyl group having 1 to 12 carbon atoms; and
a step of removing a substituent on a nitrogen atom contained in a piperidine ring in the 1-substituted-trans-4-(protected amino)piperidin-3-ol represented by formula (VI).

11. The producing method according to claim 10,
wherein R³ in formulas (VI) and (VII) is a tert-butyl group.

12. The producing method according to any one of claims 1 to 3, wherein the compound represented by formula (II) is a compound represented by formula (II-D): 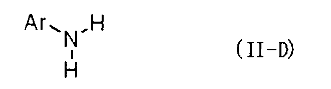 wherein Ar represents a phenyl group which may be substituted, and the substituent is one or more substituent(s) selected from the group consisting of an alkyl group having 1 to 12 carbon atoms, an alkoxy group having 1 to 12 carbon atoms, a halogen atom, a protected amino group and a protected hydroxyl group, and the compound represented by formula (III-1) is a compound represented by the formula (III-D): 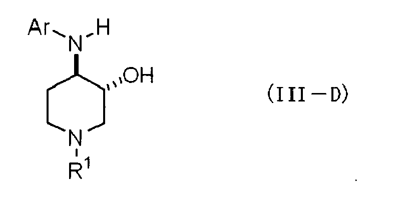 wherein R¹ and Ar are respectively as defined above.

13. The producing method according to claim 12, wherein R¹ in formula (III-D) is a benzyl group.
